# EUROPEAN PATENT SPECIFICATION

(11) **EP 2 716 263 B1**
(45) Date of publication and mention of the grant of the patent: **14.12.2016**
(21) Application number: 12789310.5
(22) Date of filing: 23.05.2012
(51) Int. Cl.: A61F 2/82, A61L 27/04, A61M 29/02, A61M 25/01, A61F 2/90, A61F 2/915, A61B 17/12

(54) **STENT FOR THE COIL EMBOLIZATION OF A CEREBRAL ANEURYSM**
STENT ZUR SPULENEMBOLISIERUNG EINES HIRNANEURYSMAS
ENDOPROTHÈSE POUR L'EMBOLISATION PAR SPIRALE D'UN ANÉVRISME CÉRÉBRAL

(30) Priority: 26.05.2011 KR 20110050292
(43) Date of publication of application: 09.04.2014
(73) Proprietor: Dongguk University Industry-Academic Cooperation Foundation, Seoul 100-715 (KR); The Asan Foundation, Seoul 138-736 (KR)
(72) Inventor: KIM, Tae-Il, Seoul 150-755 (KR); LIM, Ok-Kyun, Seoul 136-130 (KR); YANG, In-Chul, Gyeonggi-do 472-909 (KR); KIM, Sung-Min, Goyang-si Gyeonggi-do, 411-754 (KR); HWANG, Seon-Moon, Bucheon-si Gyeonggi-do, 420-020 (KR); LEE, Deok-Hee, Seoul, 138-787 (KR)
(74) Representative: Groth & Co. KB
(86) International application number: PCT/KR2012/004066
(87) International publication number: WO 2012/161509

(56) References cited:
- WO-A1-2009/076515
- WO-A1-2010/120926
- WO-A2-00/44308
- US-A1- 2002 007 222
- US-A1- 2004 167 605
- US-A1- 2004 260 384
- US-A1- 2007 100 430
- US-A1- 2009 287 297
- US-A1- 2010 010 624
- US-A1- 2010 211 162

## Description

### Technical Field

The following description relates to a stent, and more specifically, a stent used for coil embolization of a cerebral aneurysm.

### Background Art

A cerebral aneurysm is a disorder in which weakness demage or deficit of the internal elastic lamina and the media, both of which constitute the interior of a cerebral vessel, causes the blood vessel to inflate to thereby form a space in the blood vessel. If a cerebral aneurysm is left without treatment, a thickness of a blood vessel wall gradually becomes thinner and damaged, and, at some point, may be ruptured due to a continuous pressure of blood flow. In particular, a ruptured cerebral aneurysm leads to a cerebral hemorrhage, thereby resulting in a more serious live-threatening consequence than any other aneurysm. For this reason, numerous medical technologies have been developed to treat exclusively a cerebral, apart from other types of aneurysms.

On a broad sense, there are two options for treatment of a cerebral aneurysm; clip ligation and coil embolization. Clip ligation of a cerebral aneurysm is a conventional neurosurgery way for cerebral aneurysm treatment by removing cranial bones and ligating the aneurysm with a clip. Clip embolization is performed by inserting a small metal tube through a femoral artery in a leg to reach a cerebral aneurysm, and then filling up the aneurysm with coil. Since craniotomy is not required for clip embolization, a patient may undergo the surgery for a short time and may recover and return to a normal life within few days.

In other words, coil embolization prevents blood from entering a cerebral aneurysm by filling up the aneurysm with a coil. In treatment of a cerebral aneurysm using coil embolization, about 20 % cases do not require additional ancillary devices. But, in the case of a wide neck cerebral aneurysm with a large orifice, it is necessary to insert a stent into a parent blood vessel to cover a neck of the cerebral aneurysm so as to prevent migration of a coil that fills the aneurysm. That is, the stent used for coil embolization aims to prevent migration of the packed coil, and is a mesh-structured thin metal wire through which a coil fills an aneurysm.

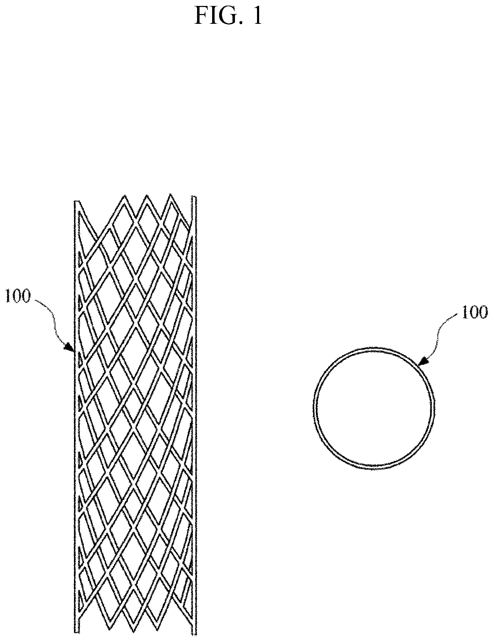

FIG. 1 is a diagram illustrating a conventional stent for coil embolization of cerebral aneurysm, including a front view (on the left-hand side) and a lateral view (on the right-hand side). Referring to FIG. 1, a stent 100 has a hollow cylindrical shape. That is, an outer circumferential surface of the stent 100 is limited by a mesh structure woven by a thin metal wire, and has an open top and a bottom top with a hollow interior. The stent 100 in a cylindrical shape has a constant diameter. As shown in the front view of the stent 100, a middle portion and two edge portions of the stent 100 have the same diameter. The stent 100 is inserted into a cerebral vessel harboring an aneurysm so as to cover a neck of the aneurysm, and a coil is inserted into the cerebral vessel through a mesh on the outer circumference surface of the stent 100.

For a common cerebral aneurysm, for example, a cerebral aneurysm with an average size neck and a cerebral aneurysm arising from a straight cerebral vessel, the conventional stent 100 is effective in preventing migration of a coil. However, a cerebral vessel has a relatively complex structure and/or shape. In addition, the complex structure and/or shape often lead to the cerebral aneurysm to have a unique shape. For example, a cerebral aneurysm may be an aneurysm which arises from a basilar artery top or from a connecting point between a cerebral vessel and any peripheral blood vessel, and/or a wide neck cerebral aneurysm with a relatively large orifice.

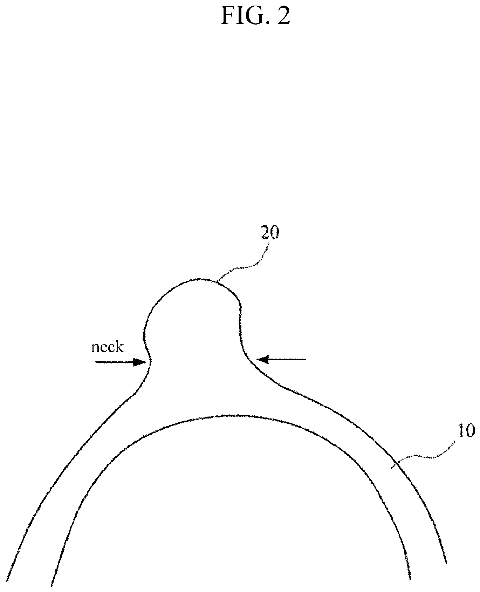

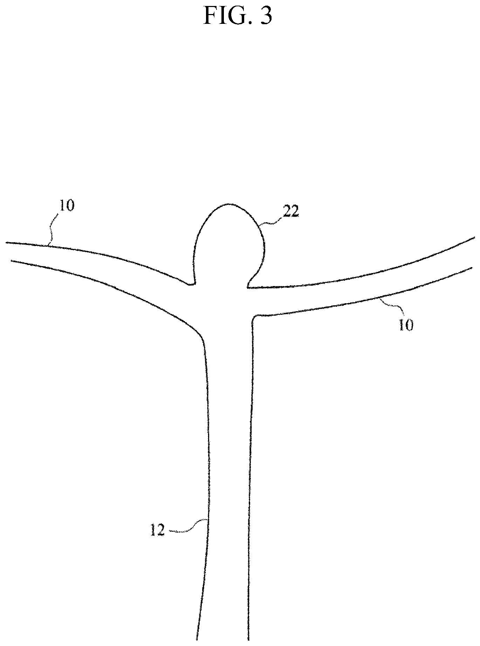

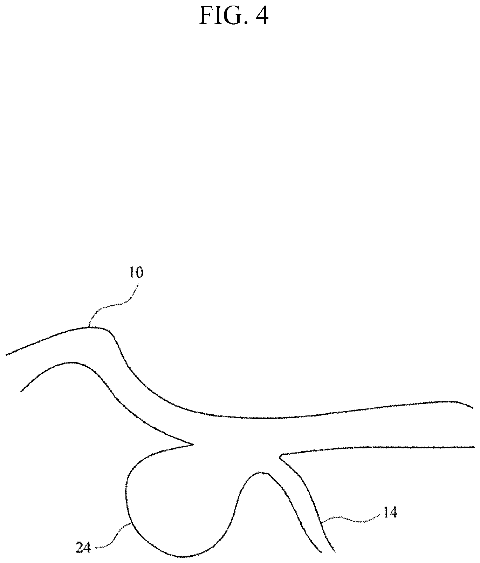

In such cases, if coil embolization is performed using the conventional stent 100 shown in FIG. 1, a coil that fills the aneurysm may subsequently fall into the cerebral vessel. FIGS. 2 to 4 are diagrams illustrating examples of a cerebral aneurysm, the aneurysm for which coil embolization is performed using the conventional stent 100, possibly leading migration of a coil: FIG. 2 is a wide neck cerebral aneurysm with a relatively large orifice, that is, a cerebral aneurysm 20 that arises from a parent artery 10, and has a relatively large orifice; FIG. 3 is a cerebral aneurysm 22 arising from a basilar artery top bifurcated into left and right parent artery 10; and FIG. 4 is a cerebral aneurysm 24 arising from a connecting point between the parent artery 10 and a bifurcated blood vessel 14. If the conventional stent 100 (See FIG. 1) is used for the cerebral aneurysms 20, 22 and 24, which are shown in FIGS. 2A to 2C, a wide gap may exist between a neck of any one of the cerebral aneurysms 20, 22 and 24 and the stent 100 due to a unique shape or a location of the cerebral aneurysm. In this case, chances are high that a coil contained in the cerebral aneurysm 20, 22 or 24 may fall into the blood vessel, and the fallen coil may cause damage to the artery 10, 12 or 14, or, in some cases, block the entire blood vessel 10, 12 or 14.

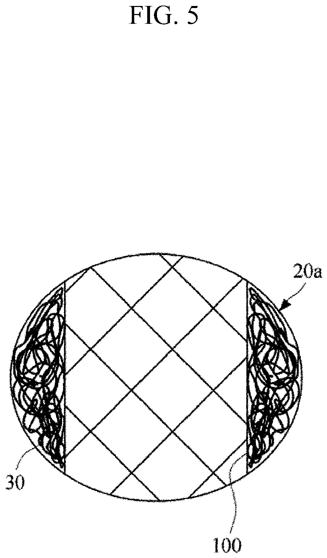

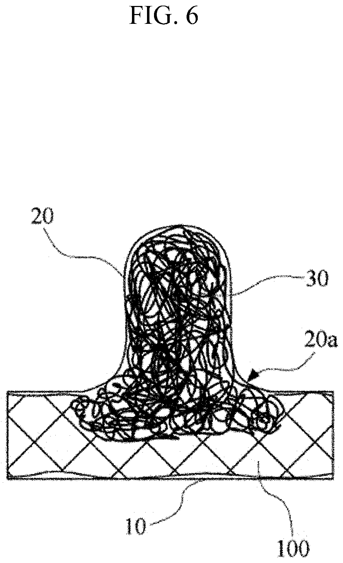

FIGS. 5 and 6 are diagrams illustrating an example in which the conventional stent 100 used for coil embolization of a cerebral aneurysm is inserted. FIG. 5 is a view from a neck 20a of the cerebral aneurysm 20, and FIG. 6 is a broad view of the stent 100 is inserted into a cerebral artery. Referring to FIGS. 5 and 6, there is a considerable wide gap between the stent 100 and a neck 20a of the cerebral artery due to a small diameter of the stent 100, so that a considerably wide gap exists between the stent 100 and the neck 20a of the cerebral aneurysm, and the chances are high that a coil falls into the cerebral vessel 10 through the gap.

US 2004/260384 A1 discloses a system for treating an aneurysm, including a catheter with a stent-receiving tube and a coiled stent axially received in the stent-receiving tube, wherein the coiled stent is delivered to the aneurysm through a vessel via the catheter.

WO 2010/120926 A1 discloses flexible implantable occluding devices that can, for example, navigate the tortuous vessels of the neurovasculature. The occluding devices can also conform to the shape of the tortuous vessels of the vasculature.

US 2002/007222 A1 discloses a structural apparatus positioned exterior of a body organ for providing support. In one configuration, the support is in the form of a strap that has a first length with holes in which a second length with saw tooth edges is inserted to form a band to surround and give support to a body organ.

### Technical Problem

The objective of the present invention is to provide a stent used for coil embolization of various cerebral aneurysm, including a cerebral aneurysm with a unique shape, such as a wide neck cerebral aneurysm with a large orifice, and a cerebral aneurysm arising from a cerebral vessel with a complex shape or structure, such as a cerebral aneurysm arising from a curvature part of a vessel, e.g., a basilar artery top, and a cerebral aneurysm arising from a connecting point between a cerebral artery and a bifurcated blood vessel.

### Technical Solution

Provided is a stent used for coil embolization of a cerebral aneurysm, configured to be in a cylindrical shape wherein a maximum diameter of a middle portion of the stent is greater than that of edge portions proximal to the middle portion, wherein the cylindrical shape is made of a mesh-structured thin metal wire so as to help a coil to fill the cerebral aneurysm through a mesh of an outer surface of the stent, wherein one side of the middle portion protrudes further than both of the edge portions, wherein at least one of the edge portions has a cylindrical shape with constant diameter and wherein the stent is curved on an opposite direction against a direction toward which the middle portion protrudes.

One protrusion markers made of radio-opacity materials may be installed at the middle portion.

The middle portion may have a length of between 4mm and 40mm. The middle portion may have a maximum diameter of between 2mm and 8mm.

### Advantageous Effects

In exemplary embodiments of the present invention, a stent used for coil embolization of cerebral aneurysm is configured to have a middle portion further protruding than edge portions thereof. Even in a case that the stent is used for a wide neck cerebral aneurysm with a relatively large orifice, a cerebral aneurysm arising from a complex structured region, such as a connection point between a cerebral artery and any other blood vessel, a gap between a neck of the cerebral aneurysm and the stent may be reduced as much as possible. Accordingly, it is possible to block or prevent migration of a coil contained in the cerebral aneurysm, and thus any side effects from the coil's falling into a blood vessel may be prevented. In addition, due to one or more protruding markers disposed on the middle portion, the stent may be placed such that the middle portion is directly on the neck of the cerebral aneurysm when coil embolization is performed.

### Description of Drawings

The accompanying drawings, which are included to provide a further understanding of the invention and are incorporated in and constitute a part of this specification, illustrate embodiments of the invention, and together with the description serve to explain the principles of the invention.
FIG. 1 is a front view and a lateral view of a conventional stent used for coil embolization of a cerebral aneurysm.
FIGS. 2 to 4 illustrate examples of a cerebral aneurysm, for which the stent shown in FIG. is used, possibly resulting in a problem: FIG. 2 is an example of a wide-neck cerebral aneurysm with a relatively large orifice; FIG. 3 is an example of a cerebral aneurysm arising from a basilar artery top; and FIG. 4 is an example of a cerebral aneurysm arising from a connecting point between a parent artery and a bifurcated blood vessel.
FIGS. 5 and 6 illustrating an example in which the stent is inserted in a cerebral vessel: FIG. 5 is a view from a neck of a cerebral aneurysm; FIG. 6 is a lateral view of a stent which is inserted in a cerebral aneurysm.
FIG. 7 is a front view of a stent used for coil embolization of a cerebral aneurysm according to an example which is useful for understanding the present invention;
FIG. 8 is a diagram illustrating an example in which the stent shown in FIG. 7 is inserted into a cerebral vessel harboring a wide neck cerebral aneurysm;
FIGS. 9 and 10 are examples in which the stent shown in FIG. 7 is inserted: FIG. 9 is a view of the inserted stent from a neck of a cerebral aneurysm; FIG. 10 is a cross sectional view of the inserted stent.
FIG. 11 is a front view of a stent for coil embolization of a cerebral aneurysm according to an example which is useful for understanding the present invention.
FIG. 12 is an example in which the stent shown in FIG. 11 is inserted for coil embolization of a cerebral aneurysm arising from a connecting point between a cerebral artery and a bifurcated blood vessel.
FIG. 13 is a front view of a stent used for coil embolization of a cerebral aneurysm according to an exemplary embodiment of the present invention.
FIG. 14 is an example in which the stent in FIG. 13 is used for coil embolization of a cerebral aneurysm that arises from a point where a basilar artery is bifurcated into cerebral arteries.
FIG. 15 is a front view of a stent used for coil embolization of a cerebral aneurysm according to another exemplary embodiment of the present invention.

### Detailed Description of the Invention

The invention is described more fully hereinafter with reference to the accompanying drawings, in which examples of the invention are shown. In the drawings, the size and relative sizes of layers and regions may be exaggerated for clarity. Like reference numerals in the drawings denote like elements.

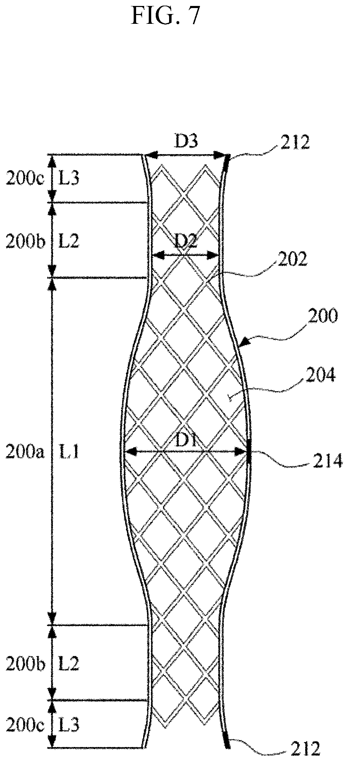

FIG. 7 is a front view of a stent used for coil embolization of a cerebral aneurysm according to an example which is useful for understanding the present invention.

Referring to FIG. 7, a stent 200 is a cylinder-shaped and mesh-structured thin metal wire 202 in a cylinder shape. An outer circumferential surface of the stent 200 is limited by the mesh-structured thin metal wire, but both edge portions thereof are open. A plurality of empty spaces 204 (corresponding to meshes) are formed on the outer surface of the stent 200. Such empty spaces are used as a passage through which a coil is deployed from inside the stent 200 into an aneurysm when coil embolization is performed.

The stent 200 consists of a middle portion 200a and a pair of edge portions 200b and 200c, and the edge portions 200b and 200c are located at both edges of the stent 200. The middle portion 200a and the edge portions 200b and 200c may be distinguishable physically, conceptually and/or functionally. Although not illustrated in FIG. 7, an ancillary member may be may be provided in stent 200, specifically on a boundary between the middle portion 200a and each of the two edge portions 200b and 200c to distinguish the middle portion 200a and each of the two edge portions 200b. For example, if the stent 200 is in a cylindrical shape with a protruding central part, the middle portion 200a may be a protruding portion including the central part of the stent 200, and the edge portions 200b and 200c may be both edges of the middle portion 200a. Alternatively, if the stent 200 is inserted into a cerebral artery harboring a cerebral aneurysm, a central part of the stent 200, which is big enough to cover a neck of the cerebral aneurysm, may be the middle portion 200a and the rest of the stent 200 may be the edge portions 200b and 200c.

In one example, the edge portions 200b and 200c may consist of a first edge portion 200b proximal to the middle portion 200a, and a second edge portion 200c, which is on the outer side of the first edge portion 200b, that is, a part distal from the middle portion 200a. For example, as illustrated in FIG. 7, if the stent 200 is in a fallopian-tube form such that a inner part (that is, a part proximal to the middle portion 200a) of the first and second edge portions 200b and 200c has a greater diameter than a outer part thereof, the inner part corresponds to the first edge portion 200b and the outer part corresponds to the second edge portion 200c. As such, the first and second edge portions 200b and 200c may be distinguishable physically, but aspects of the present invention are not limited thereto. In another example described in the following, in which the stent 200 is not in a fallopian-tube form and the edge portions 200b and 200c has the same diameter, the first and the second edge portions 200b and 200c may not be distinguishable physically.

In the above example, the stent 200 is characterized in that a maximum diameter D1 of the middle portion 200a is greater than a maximum diameter D2 of the first edge portion 200b. Herein, each of the maximum diameters D1 and D2 refers to the greatest diameter of a corresponding portion. For example, the stent 200 is fusiform in shape such that the middle portion 200a protrudes further than the first edge portion 200b so that the maximum diameter D1 of the middle portion 200a may be greater than the maximum diameter D2 of the first edge portion 200b.

In the case where the stent 200 is a fallopian-tube shape, the maximum diameter D1 of the middle portion 200a may be greater than a maximum diameter D3 of the second edge portion 200c. However, aspects of the present invention is not limited thereto, and the maximum diameter D1 of the middle portion 200a may be equal to or smaller than the maximum diameter D3 of the second edge portion 200c.

In another example, the stent 200 may have various profiles so that the maximum diameter D1 of the middle portion 200a may be greater than the maximum diameter D1 of the first edge portion 200b. That is, the stent 200 may have various profiles while satisfying the above-described condition (D1>D2). For example, the stent 200 may have a profile in which a diameter gradually increases from the second edge portion 200c through the first edge portion 200b to the middle portion 200a. In another example, the stent 200 may have a profile in which a diameter is constant for the first and second edge portions 200b and 200c, but gradually increases toward a central part of the middle portion 200a. Specifically, a diameter in the middle portion 200a gradually increases toward a central part thereof so that a maximum diagram is achieved at the central part, or a maximum diameter of the middle portion 200a may be maintained for a specific width of the middle portion 200a (that is, a profile in which the farthest protruding central part of the middle portion 220a is flat).

As such, the stent 200, having a profile in which the middle portion 200a that protrudes further than at least the first edge portion 200b, is inserted into a cerebral vessel harboring a cerebral aneurysm, a gap between a neck of the cerebral aneurysm and the stent 200 may be eliminated or minimized, so that it is possible to prevent or minimize migration of a coil contained in the cerebral aneurysm. In particular, for a wide neck cerebral aneurysm (See FIG. 2) or a cerebral aneurysm arising from a uniquely shaped or structured blood vessel (See FIGS. 3 and 4), the stent 200 may be used more effectively to prevent migration of a coil.

As described above, the stent 200 may be limited by a mesh-structured thin metal wire 202. That is, the stent 200 may be fusiform by weaving the thin metal wire 202 in a lattice structure. An empty space 204 (corresponding to a mesh) limited by the lattice structure may be rhombus, but aspects of the present invention is not limited thereto. That is, the empty space 204 may have various shapes as long as it is large enough to perform a coil embolization. For example, the empty space 204 may have an area (for example, an area greater than 1mm) through which a micro catheter used for coil embolization, that is, a micro catheter having a diameter smaller than 1mm, is able to pass easily. The lattice-structured thin metal wire 202 may be closed such that edges of neighboring meshes are connected to each other (See FIG. 7) or may be open such that edges of some meshes are not connected to each other.

As such, the stent 200 has the first edge portions 200b positioned on both ends of the middle portion 200a, and the maximum diameter D2 of the first edge portions 200b is smaller than the maximum diameter D1 of the middle portion 200a. While satisfying the above condition (D1>D2), the first and second edge portions 200b and 200c may have the same diameter or a profile in which the diameter of the first and second edge portions 200b and 200c gradually decreases in a distal direction toward the middle portion 200a. Alternatively, as illustrated in FIG. 7, the stent 200 may be in a fallopian-tube form such that a diameter of the first and second edge portions 200b and 200c gradually increase in a distal direction to the middle portion 200a. In the case where the stent 200 in the fallopian-tube form is inserted into a blood vessel, the stent 200 may conforms to the inner wall of the artery so as to be securely fixed at a desired location inside the cerebral artery.

The stent 200 may include end markers 212, and each of the end markers 212 is installed at the margin of the edge portions 200b and 200c of the stent 200, specifically at the second edge portions 200c. One or two end markers 212 may be provided, and each end marker 212 is usually made of radio-opacity materials. Using the end markers 212 disposed on the second edge portions 200c of the stent 200, a practitioner may easily find out both ends of the stent 200, that is, a distal part and a proximal part of the stent 200, which are inserted into a blood vessel under X-ray.

In an example, the stent 200 may further include a protrusion marker 214 in the middle portion 200a as well as the end markers 212. One or more protrusion markers 214 may be provided, but FIG. 7 illustrates an example in which only one protrusion marker 214 is provided. The protrusion marker 214 is informs a practitioner of the location of an area with a maximum diameter in the middle portion 200a of the stent 200. Thus, using only one protrusion marker 214 disposed at a part with the maximum diameter D1, as illustrated in FIG. 7, or using a plurality of protrusion markers 214, for example, adding two additional protrusion markers symmetrically on the left and right side of the protrusion marker 214 in FIG. 7, a practitioner may easily find a location of an exceptionally protruding part of the middle portion 200a. Since the protrusion marker 214 is used to help a corresponding part (a protrusion part) thereof to be placed on a neck of a cerebral aneurysm, the protrusion marker 214 may be utilized more efficiently for treatment of a wide neck cerebral aneurysm or a cerebral aneurysm that arises from a uniquely shaped or structured blood vessel.

In an example, the maximum diameter D1 of the middle portion 200a may be between 2.5mm and 8mm. In addition, the length of the middle portion 200a may be between 4mm and 30mm. Having the maximum diameter D1 and the length as specified above, the middle portion 200a may be a symmetric fusiform with a gentle or steep slope. Taking into account an internal diameter of a cerebral artery harboring a cerebral aneurysm, a length L2+L3 of the first and second edge portions 200b and 200c of the stent 200 may be between 2mm and 6mm. In addition, the whole length L1+2×(L2+L3) of the stent 200 is a sum of the length of the middle portion 200a and the length of the edge portions 200b and 200c, and the length L1+2×(L2+L3) may be between 10mm and 40mm.

The thin metal wire 202 of the stent 200 configured as above may be shape-memory alloy. Shape-memory alloy is usually made of nitinol, but aspects of the present invention are not limited thereto. Nitinol is a metal alloy of nickel and titanium. Characterized by a crystal structure that is changeable according to temperature, a shape of shape-memory alloy may be changed into any other shape at low temperatures but, if temperatures are raised, may revert to the original shape. If reverting to the original shape, properties of shape-memory alloy may become much stronger. Due to the characteristic of shape-memory alloy, the stent 200 maintains its small size at room temperatures for easy insertion into an artery, however, when inserted into a blood vessel, temperature changes may cause the stent to self-expand and conform to the inner wall of the blood vessel.

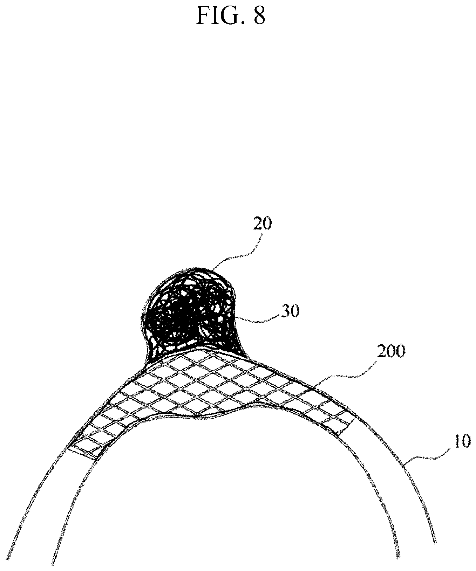

FIG. 8 is a diagram illustrating an example in which the stent 200 shown in FIG. 7 is inserted into a cerebral vessel 10 harboring a wide neck cerebral aneurysm. For convenience of explanation, FIG. 8 demonstrates the stent 200 with edge portions with a constant diameter, and end markers and protrusion markers are not omitted in FIG. 8. FIG. 8 relates to an example in which a cerebral aneurysm 20 is filled with a coil 30 by performing coil embolization.

Referring to FIG. 8, the stent 200 used for coil embolization of a cerebral aneurysm is inserted into a cerebral artery 10 harboring the cerebral aneurysm 20. In particular, the middle portion 200a (See FIG. 7) of the stent 200 is located inside the cerebral vessel 10 to cover at least the neck of the cerebral aneurysm 20. If the cerebral aneurysm 20 arising from the cerebral artery 10 is a wide neck cerebral aneurysm, it is hard for the conventional stent 100 (See FIG. 1) to block a neck of the cerebral aneurysm 20 so that a relatively large orifice may occur between the stent 100 and the neck of the cerebral aneurysm 20 (See FIG. 5). By contrast, if the stent 200 is used, it is possible to effectively block even a neck of a wide neck cerebral aneurysm since the stent 200 is a fusiform shape with the middle portion 200a (See FIG. 7) protruding further than the first edge portion 200b (See FIG. 7) so that the middle portion 200a fully covers the neck of the cerebral aneurysm. Therefore, if the stent 200 is used for coil embolization, it is possible to effectively prevent migration of a coil contained in the cerebral aneurysm.

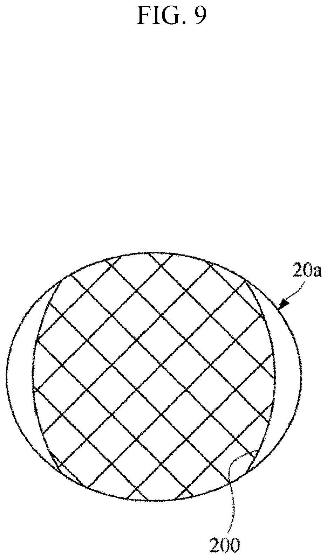

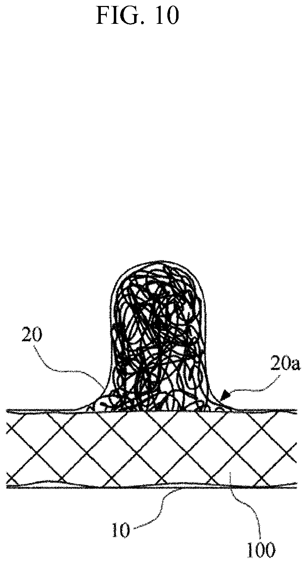

FIGS. 9 and 10 are examples in which the stent 200 is inserted: FIG. 9 is a view of the inserted stent 200 from a neck 20a of the cerebral aneurysm 20; and FIG. 10 is a cross sectional view of the inserted stent 200. Referring to FIGS. 9 and 10, the stent 200 is fusiform such that a middle portion of the stent 200 has a diameter greater than that of an edge portion, and thus, a gap hardly occurs between the stent 200 and the neck 20a of the cerebral aneurysm. Therefore, the stent 200 may help to significantly reduce the possibility of a coil 30 contained in the wide neck cerebral aneurysm 20 falling into the cerebral vessel 10.

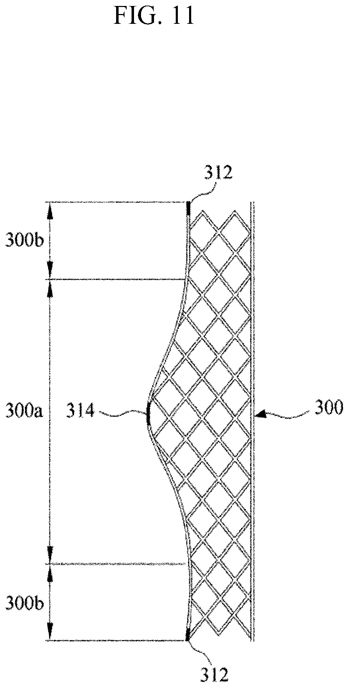

FIG. 11 is a front view of a stent for coil embolization of a cerebral aneurysm according to an example which is useful for understanding the present invention. Hereinafter, differences from the stent 200 will be mainly described with reference to FIG. 7. Descriptions not provided in the following may be the same as described in the above with respect to the stent 200. In the following example, there is provided a stent, rather than being in a fallopian-tube form, with a profile such that an edge portion has a constant diameter, but it does not mean that a possibility of being in the fallopian-tube form is excluded. Therefore, an 'edge portion' in the following example indicates all the parts (that is, the first and second edge portions in FIG. 7) of the stent, except for a 'middle portions.'

Referring to FIG. 11, a stent 300 includes a middle portions 300a and both edge portions 300b, as the same as the stent 200 in FIG. 7. In addition, as shown in FIG. 11, a maximum diameter of the middle portion 300a is greater than that of an edge portion 300b. However, the stent 300 is different from the stent 200 in FIG. 7 since the middle portion 300a is not an entirely protruding fusiform, but a semi-fusiform with one protruding side (the right side in FIG. 11) and one straight side (the left side in FIG. 11) toward the edge 300b. Further, the stent 300 may include a protrusion marker 314 as well as end markers 312, and the protrusion marker 314 of the stent 300 in FIG. 11 may indicate an accurate location of a protruding part and a protruding direction of the stent 300.

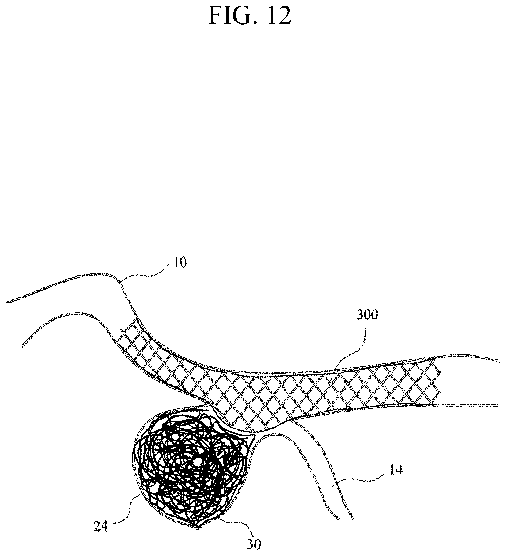

FIG. 12 is an example in which the stent 300 in FIG. 11 is used for coil embolization of a cerebral aneurysm, and specifically, an example in which the stent 300 is used for coil embolization of a cerebral aneurysm arising from a connecting point between a cerebral artery and a peripheral blood vessel. Referring to FIG. 12, a cerebral aneurysm 24 arising from a connecting point between a cerebral artery 10 and a bifurcated blood vessel 12 thereof may have a relatively wide neck. In this case, if coil embolization is performed using the conventional linear-type stent 100 (See FIG. 1), the stent 100 may not fully make contact with a neck of the cerebral aneurysm 24 due to the unique shape of the connecting point between of the cerebral vessel 24 and the bifurcated blood vessel 12. On the other hand, if coil embolization is performed using the stent 200, the neck of the cerebral aneurysm 24 may be blocked effectively, but the inner wall of the cerebral aneurysm 24 on the opposite side of the cerebral aneurysm 24 may be pressed by the stent 200. For this drawback, the semi-fusiform stent 300, shown in FIG. 11, is used to reduce a gap between the stent 300 and the neck of the cerebral aneurysm 24, and to reduce pressure on the inner wall of the cerebral aneurysm 24 on the opposite side of the cerebral aneurysm 24.

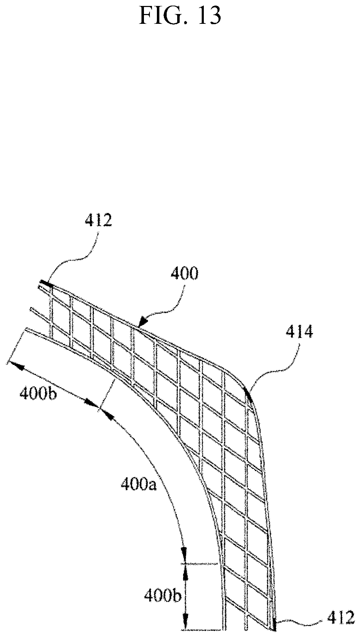

FIG. 13 is a front view of a stent used for coil embolization of a cerebral aneurysm according to one exemplary embodiment of the present invention. Hereinafter, differences from the stents 200 and 300 will be mainly described with reference to FIGS. 7 and 11. Descriptions not provided in the following may be the same as described above with respect to the stents 200 and 300 with reference to FIGS. 7 and 11.

Referring to FIG. 13, a stent 400 includes a middle portion 400a and both edge portions 400b, as the same as the stents 200 and 300 in FIGS. 7 and 11, respectively. In addition, as shown in FIG. 13, a maximum diameter of the middle portion 400a is greater than that of an edge portion 400b. Just like the stent 300 in FIG. 11, the middle portion 400a is not an entirely-protruding fusiform, but a semi fusiform with one protruding side (the right side in FIG. 11) and one straight side (the left side in FIG. 11) toward the edge portions 300b. However, the stent 400 is different from the stent 300 in FIG. 11 since the stent 400 has a profile to be curved at a predetermined angle, for example, between 10 and 90 degrees, on the middle portion 400a. Nonetheless, in that the stent 400 may include the protrusion marker 414 which is capable of indicating a location of a protruding part and a protruding direction of the middle portion 400a, the stent 400 is the same as the stent 300 in FIG. 11.

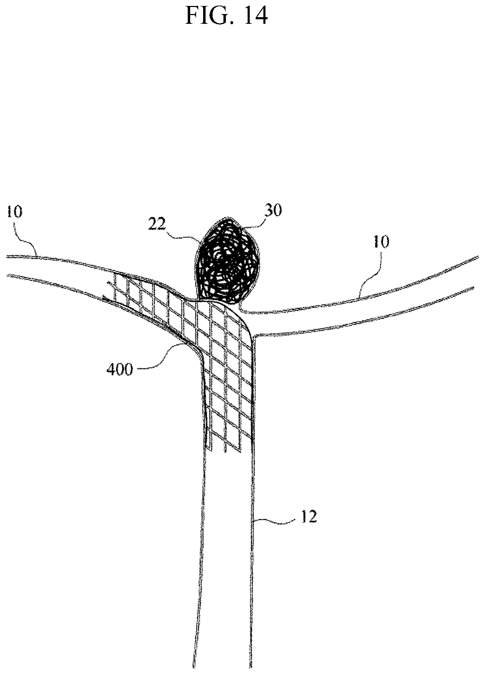

FIG. 14 is an example in which the stent 400 in FIG. 13 is used for coil embolization of a cerebral aneurysm, and more specifically, an example in which a cerebral aneurysm arises from a branch point where a basilar artery is bifurcated into cerebral artery 10. Referring to FIG. 14, a cerebral aneurysm 22 arising from a branch point where a basilar artery 12 is bifurcated into cerebral vessels 10 may have a relatively wide neck. In this case, the conventional linear-type stent 100 (See FIG. 1) may not fully contact the wide neck of the cerebral aneurysm 22. Even using the semi-fusiform stent 300 shown FIG. 11, it is hard to block the neck of the cerebral aneurysm effectively due to the complex structure of blood vessels 10 and 12. However, if the curved semi-fusiform stent 400 is used, it is possible to effectively reduce a gap between the stent 400 and the neck of the cerebral aneurysm 22.

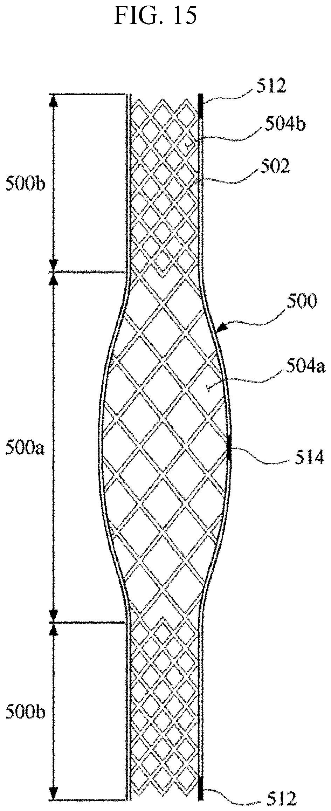

FIG. 15 is a front view of a stent used for coil embolization of a cerebral aneurysm. Hereinafter, differences from the stents 200, 300 and 400 will be mainly described with reference to FIGS. 7, 11 and 13. Thus, description not provided herein may be the same as described in the above with respect to the stents 200, 300 and 400 with respect to FIG. 7, 11 and 13.

Referring to FIG. 15, a stent 500 includes a middle portion 500a and both edge portions 500b, as the same as the above-described stents 200, 300 and 400. In addition, as shown in FIG. 15, a maximum diameter of the middle portion 500a is greater than that of an edge portion 500b. Further, just like the stent 200, the middle portion 500a is entirely-protruding fusiform. The stent 500 may include a protrusion maker 514 as well as end markers 512, and the protrusion maker 514 may indicate a location of a protruding part and a protruding direction of the middle portion 500a.

The stent 500 in FIG. 15 is different from the stent 200 in FIG. 7 since there is a difference in a size of an empty space limited by a mesh-structured thin metal wire 502 between the middle portion 500a and the edge portions 500b. Specifically, the stent 500 is configured that the thin metal wire 502 is more densely woven at the edge portions 500b than at the middle portion 500a, so that a size of a mesh 540b of an edge portion 500b is smaller than that of a mesh 504a of the middle portion 500a. As the stent 500 is configured as above, each edge portion 500b may conform to a blood vessel wall with greater force than the middle portion 500a while or after the stent 500 expands inside a blood vessel, thereby efficiently preventing migration of the stent 500 in the vessel.

### Industrial Applicability

The present invention may be used in medical device related industries.

## Claims

1. A stent (400) used for coil embolization of a cerebral aneurysm, configured to be in a cylindrical shape wherein a maximum diameter of a middle portion (400a) of the stent is greater than that of edge portions (400b) proximal to the middle portion:
wherein the stent is made of a mesh-structured thin metal wire so as to help a coil (30) to fill the cerebral aneurysm through a mesh of an outer surface of the stent,
wherein one side of the middle portion (400a) protrudes further than both of the edge portions (400b),
wherein at least one of the edge portions (400b) has a cylindrical shape with constant diameter, and
wherein the stent is curved on an opposite direction against a direction toward which the middle portion (400a) protrudes.

2. The stent of claim 1, wherein one protrusion markers (414) made of radio-opacity materials are installed at the middle portion.

3. The stent of any one of claim 1 or 2, wherein the middle portion (400a) has a length of between 4mm and 40mm.

4. The stent of any one of claims of 1 to 3, wherein the middle portion (400a) has a maximum diameter of between 2mm and 8mm.

## Patentansprüche

1. Stent (400) zur Verwendung für die Coilembolisation eines Hirnaneurysmas, der dazu konfiguriert ist, in einer zylindrischen Form zu sein, wobei ein maximaler Durchmesser eines mittleren Abschnitts (400a) des Stents größer als der von proximal zum mittleren Abschnitt liegenden Randabschnitten (400b) ist,
wobei der Stent aus einem dünnen Metalldraht mit Netzstruktur hergestellt ist, um eine Spirale (30) dabei zu unterstützen, das Hirnaneurysma durch ein Netz einer Außenfläche des Stents zu füllen,
wobei eine Seite des mittleren Abschnitts (400a) weiter als beide Randabschnitte (400b) vorragt, wobei mindestens einer der Randabschnitte (400b) eine zylindrische Form mit konstantem Durchmesser hat und
wobei der Stent in einer Richtung gekrümmt ist, die der Richtung, in die der mittlere Abschnitt (400a) vorragt, entgegengesetzt ist.

2. Stent nach Anspruch 1, wobei eine Vorsprungsmarkierungen (414), die aus röntgenopaken Materialien hergestellt sind, am mittleren Abschnitt angebracht sind.

3. Stent nach einem der Ansprüche 1 oder 2, wobei der mittlere Abschnitt (400a) eine Länge zwischen 4 mm und 40 mm hat.

4. Stent nach einem der Ansprüche 1 bis 3, wobei der mittlere Abschnitt (400a) einen maximalen Durchmesser von zwischen 2 mm und 8 mm hat.

## Revendications

1. Stent (400) utilisé pour l'embolisation par coil d'un anévrisme cérébral, configuré pour être de forme cylindrique, un diamètre maximal d'une partie médiane (400a) du stent étant supérieur à celui de parties d'extrémité (400b) adjacentes à la partie médiane,
le stent étant constitué d'un fil métallique fin formant une structure maillée de façon à faciliter le remplissage de l'anévrisme cérébral par un coil (30) à travers une maille d'une surface extérieure du stent,
un côté de la partie médiane (400a) faisant davantage saillie que les deux parties d'extrémité (400b),
au moins une des parties d'extrémité (400b) présentant une forme cylindrique à diamètre constant, et
le stent étant incurvé dans une direction opposée à une direction dans laquelle la partie médiane (400a) fait saillie.

2. Stent selon la revendication 1, des marqueurs de saillie (414) constitués de matériaux radio-opaques étant installés au niveau de la partie médiane.

3. Stent selon l'une quelconque des revendications 1 et 2, la partie médiane (400a) présentant une longueur comprise entre 4 mm et 40 mm.

4. Stent selon l'une quelconque des revendications 1 à 3, la partie médiane (400a) présentant un diamètre maximal compris entre 2 mm et 8 mm.
